# EUROPEAN PATENT SPECIFICATION

(11) **EP 2 455 128 B1**
(45) Date of publication and mention of the grant of the patent: **12.08.2015**
(21) Application number: 11188928.3
(22) Date of filing: 14.11.2011
(51) Int. Cl.: A61M 25/00, A61F 2/966

(54) **Introducer assembly and sheath therefor**
Inserteranordnung und Schaft dafür
Ensemble d'introducteur et sa gaine

(30) Priority: 18.11.2010 GB 201019519
(43) Date of publication of application: 23.05.2012
(73) Proprietor: Cook Medical Technologies LLC, Bloomington, IN 47404 (US)
(72) Inventor: Rasmussen, Erik, DK-4200 Slagelse (DK); Oehlenschlaeger, Bent, DK-4623 Ll. Skensved (DK)
(74) Representative: Georgiou, Sarah Caroline

(56) References cited:
- EP-A2- 0 773 037
- WO-A1-2007/084547
- US-B1- 6 428 566

## Description

### Technical Field

The present invention relates to an introducer assembly and sheath therefor, as well as to a method of making a sheath for an introducer assembly.

### Background Art

Introducer assemblies are commonly used for performing medical treatments endoluminally. For instance, they may be used to implant into the vasculature of a patient medical devices such stents, stent grafts, vena cava filters, occlusion devices, embolisation coils and so on. Introducer assemblies may also be used for the deployment of medical instruments to carry out medical procedures, for instance within the vasculature or to specific organs of the patient.

Typical introducer assemblies are relatively very long and flexible. For instance, an assembly used for deploying a medical device within the aorta may have length in excess of one metre. Assemblies for other applications, for example for cerebral applications, may be considerably shorter but are still very long in comparison to their diameters.

Most if not all introducer assemblies have limitations in terms of the outer diameter of the assembly. This is in part to ensure that the assembly can pass easily through a patient's vasculature and also to ensure that the assembly remains sufficiently flexible so as to be able to track the path through a patient's vessels and in such a manner as not to cause damage to these during the procedure. It is common to use a guide wire to guide the introducer assembly through the patient's vasculature, in which case the introducer assembly needs to be sufficiently flexible so as to curve with the curvature of the guide wire.

This requirement of the assembly to be able to flex and curve within a patient's vasculature is often referred to as the trackability of the assembly.

During the insertion of an introducer assembly into a patient's vasculature, the assembly often rotates as it moves into and through the vessels. Given, also, the often tortuous path though which the introducer assembly has to move, this is generally made to have a round cross-section so as to exhibit similar flexibilities in bending in all rotational directions of the assembly.

As a result of the structure and stresses imposed upon the assembly, there is generally exhibited twisting of the assembly between its proximal and distal ends. Such twisting can, furthermore, result in relative twisting or rotation between the various elements of the introducer assembly. This can cause difficulties in terms of maintaining the position and integrity of the implantable medical device carried within the introducer assembly. In particular, if the carrier element upon which the medical device is held within the introducer assembly twists or rotates relative to the outer cover sheath, this can affect the state of the medical device by causing it to twist or deform to an extent which makes it unusable. This can, in a worse case situation, result in an abortive medical procedure. If a stent or stent graft twists on the introducer, it may not deploy properly when this is released from the assembly and possibly even become damaged.

In addition to the above problem, twisting of the introducer assembly during the deployment procedure creates difficulties with the alignment of a medical device carried on the introducer. This causes difficulties in particular in deployment of medical devices which require a particular rotational alignment such as, for instance, fenestrated or branched stent grafts, bifurcated stents or stent grafts and so on. These problems can be mitigated to some extent by providing on the devices radio opaque markers. These markers can assist a clinician in seeking to align the medical devices by using imaging during the deployment procedure. Even so, this is in some instances not a particularly simple procedure given the rotational flexibility of the introducer assembly, which can make fine adjustments of the rotational position of the distal end of the introducer assembly difficult to achieve in practice.

### Disclosure of The Invention

The present invention seeks to provide an improved introducer assembly, an improved sheath therefor and an improved method of manufacturing a sheath for an introducer assembly.

According to the present invention, there is provided an introducer assembly including a flexible elongate sheath element having proximal and distal ends; at least one flexible elongate carrier element having proximal and distal ends located within the sheath element; the elongate sheath element being provided an internal lumen wall, which lumen wall has a non-round cross-section extending between substantially the proximal and distal ends of the sheath element; the elongate carrier element having an external wall surface with a non-round cross-section, the internal lumen wall of the sheath element and the outer wall surface of the carrier element being of cooperating shapes, wherein the elongate sheath element and the elongate carrier element are rotationally fixed relative to one another substantially along the entirety of their lengths between said proximal and distal ends.

It is to be understood that the cross-sections refer to axial cross-sections, that is transverse to the longitudinal axis of the sheath and carrier elements.The sheath is flexible over the portion thereof with non-round cross-sections.

The complementary cross-sections of the sheath and carrier elements have a flattened configuration.

In the preferred embodiment, the internal lumen wall of the sheath element and the outer wall surface of the carrier element have shapes which are continuous between said proximal and distal ends. In other words, they have the same cross-sectional shapes throughout substantially the entirety of their lengths. This facilitates manufacture of the elements forming the assembly but it is not excluded that the assembly could have different cross-sectional shapes at different longitudinal portions thereof, for example to be oval for a certain length thereof and then to have, for example, a key element such as ribbing or the like.

In the preferred embodiment, the internal lumen wall of the sheath element is oval in transverse cross-section and the outer wall of the carrier element is similarly oval in transverse cross-section.

Advantageously, the internal wall lumen on the outer wall surface are of complimentary shapes. The elongate carrier element is preferably a close fit within the lumen of the sheath element.

The carrier element may be a carrier catheter or cannula having at its distal end a location for holding an implantable medical device. The carrier element can be a conventional pusher element. The holding location may be a zone of reduced outer diameter and/or provided with one or more device restraining elements such as holding caps, trigger wire securing features and so on. These elements are well known in the art.

The structure of outer sheath and carrier element taught herein ensures that there can be no relative rotation between the sheath and the carrier element, the preferred embodiment ensuring no such rotation for the entirety of the length of these two elements. As a result of this, if the introducer assembly is rotated or twisted during its deployment procedure, it will tend to rotate substantially along the entirety of its length thereby keeping the distal and proximal ends in much closer rotational alignment compared to conventional introducer assemblies. Moreover, any such twisting of the introducer assembly, even if it causes relative rotation between the proximal and distal ends of the assembly, will not cause rotation of the carrier element relative to the sheath element, thereby preventing or substantially reducing the risk of twisting or otherwise deforming the implantable medical device carried in the assembly.

The structure also provides another important advantage compared to prior art introducer assemblies. In structures in which it is possible to rotate one component of the assembly relative to another, for instance the outer sheath and the carrier element, the torque strength of the assembly will be dependent upon the torque strength of the strongest component of the assembly (for example the sheath or carrier element). Increasing the torque resistance of any of these elements will generally lead to a decrease in the longitudinal flexibility of the assembly, with a result that trackability is reduced. On the other hand, with the structure taught herein, the torque strengths of the sheath element and carrier element are in effect added to one another since they rotate together and cannot rotate separately. Therefore, the torque strength of the entire assembly is increased without a disadvantageous reduction in the flexibility or trackability of the introducer.

In an embodiment, the sheath element has a shape in axial cross-section which could be described as an oval ring. The carrier element is, correspondingly, oval in its outer shape (the carrier element would typically have one or more lumens within it). A structure of this nature, that is one which could be described as flattened, causes the structure to have different flexibilities in different rotational directions. In particular, when flexed about its narrower region, the assembly will be more flexible, whereas when flexed about its wider direction, it will be less flexible. It has been found that this difference in flexibilities, which is not experienced in prior art devices, can facilitate the passage of the introducer assembly through a patient's vasculature, as it enables the clinician to rotate the assembly to change its relative flexibility and thus to assist in flexing the distal end of the introducer as it passes through curves and bends in the vessels of a patient. This improved trackability is considered to be a particular advantage of this structure.

This structure, and indeed all of the structures disclosed herein which provide different flexibilities in different radial directions of the introducer assembly, provides another important advantage, particularly in the deployment of medical devices or instruments at or proximate a curve in a vessel of a patient. In a curve of a vessel, the introducer assembly will tend to rotate in such a manner that it curves about its more flexible rotational orientation, in the case of an oval structure, about one of its flattened sides. Thus, in this particular example, the introducer assembly will tend to self-orient when in a curve in only two positions, equivalent to the two flattened sides of the assembly. This self-orientation can be particularly useful in the deployment of medical devices or instruments which are rotationally dependent, for instance in the deployment of fenestrated, branched or bifurcated medical devices or in the deployment of instruments which must be oriented in a particular direction. It will be appreciated that such a device would be loaded onto the introducer assembly in appropriate rotational alignment with the non-round cross-section of the carrier element and sheath, and in such a manner that when the assembly is located at the target site within the patient, this will self-orient such that the medical device (or instrument) located at the distal end of the assembly will likewise be oriented in the correct orientation for the deployment of that device or medical instrument. For instance, in the case of a fenestrated or branched stent graft, the fenestration or branch would be aligned with the associated side vessel. Should the distal end of the introducer assembly be out of alignment in the vessel, this would be by 180° (in the case of an oval shape). The clinician can relatively easily correct this by twisting the proximal end of the introducer assembly to flip its distal end by 180°, thus into the correct orientation. This can considerably facilitate the deployment of rotationally dependent medical devices or medical instruments.

According to another aspect of the present invention, there is provided an elongate sheath element for an introducer assembly, the sheath element having an internal lumen wall which is non-round in axial cross-section, there being provided within the sheath element at least one strengthening element designed to maintain the lumen wall in its non-round configuration.

Advantageously, the strengthening element is non-round in axial cross-section of the sheath element. The strengthening element is preferably made of a metal or metal alloy. In some embodiments, the strengthening element may be a braiding, a coil or at least one ring or a combination thereof. The strengthening-element may include at least one coil and at least one stabilising ring.

According to another aspect of the present invention, there is provided a method of manufacturing an elongate sheath element, including the steps of providing a mandrel of non-round shape in axial cross-section; locating at least one sheath tubing on the mandrel and fitting within the at least one tubing at least one strengthening element, which strengthening element adopts or has a non-round shape in axial cross-section, which shape substantially corresponds to the axial cross-sectional shape of the mandrel.

### Brief Description of the Drawings

Embodiment of the present invention are described below, by way of example only, with reference to the accompanying drawings, in which:
Figure 1 shows an example of introducer assembly;
Figure 2 is a cross-sectional view of an embodiment of sheath carrier element structure;
Figure 3 is a cross-sectional view of another embodiment of sheath structure;
Figure 4 is a schematic view of an embodiment of manufacturing method for forming a sheath element of the type taught herein; and
Figure 5 is a schematic diagram of another embodiment of sheath element.

### Description of the Preferred Embodiments

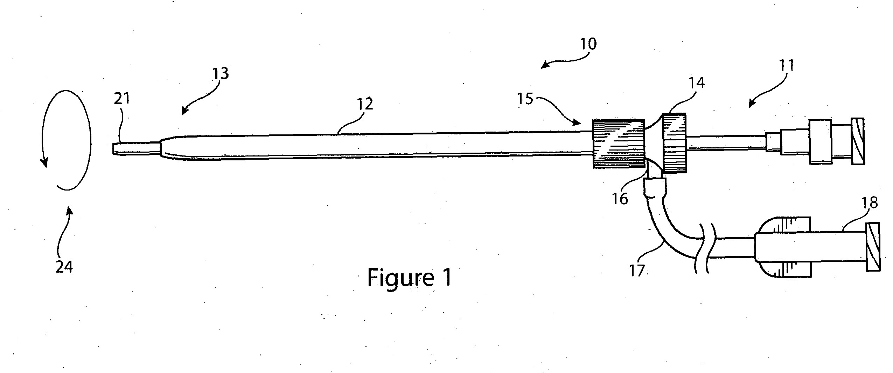

Referring to Figure 1, there is shown in schematic form an example of introducer assembly 10 for the deployment of an implantable medical device or, in some instances, for the deployment of medical instruments to be used in the carrying out of a medical procedure endoluminally within a patient. The introducer assembly 10 includes a flexible elongate sheath element 12 which extends from an external manipulation section 11 of the introducer assembly 10 towards the distal end 13 of the assembly. The components of the external manipulation assembly 11 can be of a type known in the art and are therefore not described in detail herein. They will typically include a connector valve assembly 14 carrying a lumen passing therethrough which aligns with the internal lumen of the sheath 12, a side port 16 coupled to a Luer lock connector hub 18 through coupling tubing 17.

Extending within the sheath 12, and generally movable longitudinally therein, there is provided a flexible elongate carrier element 20 which is typically in the form of a catheter or cannula. The carrier element 20 extends from a position proximal the connector valve hub 14, all the way to the distal end 13 of the sheath element 12 and in some embodiments beyond this. At the distal end of the carrier element 20 there is typically provided a dilator tip 21.

The assembly 10 and in particular the sheath 12 and carrier element 20 are typically long, particularly in comparison to their diameters. For aortic applications, for instance, the sheath 12 can be in excess of one metre in length with a diameter in the region of 30 French (10mm) or so. In other examples, for instance, cerebral, or other small lumen applications, the sheath 12 may have a much smaller diameter, for instance in the region of just a few millimetres but still have a very substantial length.

As a result of the structure of the assembly 12, and in particular the need for the sheath 12 and carrier 20 to be flexible so as to be trackable within a patient's vasculature, there is a tendency for the introducer assembly 10 to twist of rotate along its length, as shown in the arrow 24 when used. This causes the distal end 13 of the assembly 10 to rotate relative to the external manipulation end 11. As described above, this rotation can cause problems in the deployment of medical devices and medical instruments.

The embodiments of introducer assembly 10 taught herein provide for the sheath 12 and carrier element 20 to have cooperating internal and external surfaces, respectively, which are non-round in cross-section so as to cause these to be what could be described as rotationally fixed relative to one another, preferably for the entire length of the sheath element 12. In practice, this rotational fixation does not have to extend for the entirety of the length of the sheath 12 but only for a majority of its length, although it is particularly advantageous if this occurs for the entirety of the sheath 12. Such rotational fixing, as described above, can prevent or substantially reduce the rotation of the sheath 12 relative to the carrier element 20, particularly at the distal end 13. (In practice there may be a very slight rotational movement as a result of manufacturing tolerances and gaps within the assembly.)

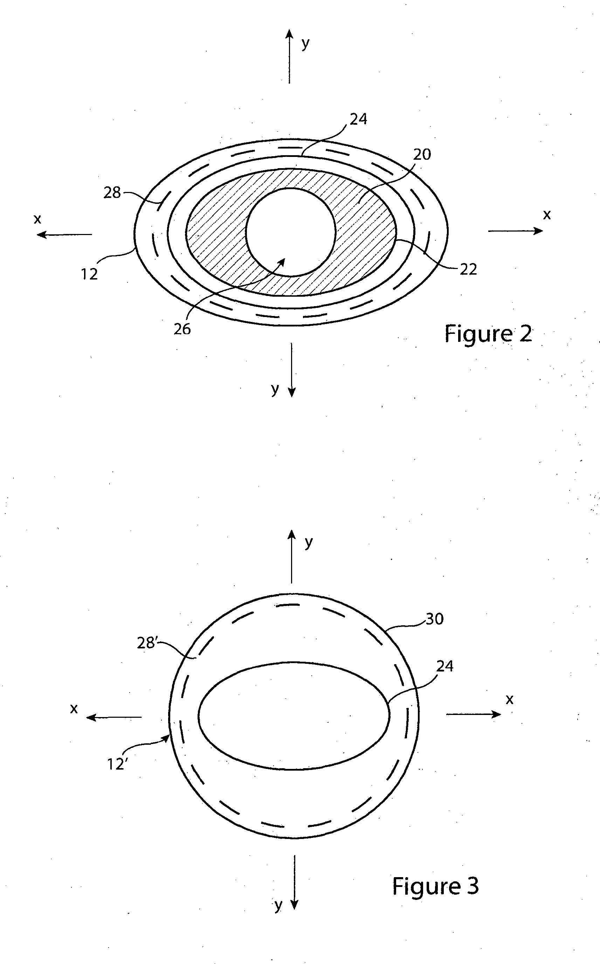

The non-round cross-section could be provided in some embodiments simply by a rib and corresponding channel on or within the sheath 12 and carrier element 20, the rib residing within the channel at all times so as to lock rotationally the two components to one another. In the preferred embodiment, however, the non-round aspect results for the shape of the internal wall of the sheath element 12 and of the external or outer wall surface of the carrier element 20. Particularly preferred embodiments are shown in Figures 2 and 3.

Referring to Figure 2, this shows a first embodiment of structure for the sheath 12 and carrier element 20, this being a cross-sectional view along the line AA of Figure 1. As can be seen, the sheath 12 has a shape which can be described as an oval ring, such that the sheath 12 is externally oval, as well as be an internally oval. The carrier element 20 has an outer wall 22 which is oval and which corresponds substantially to the shape of the internal wall 24 (the luminal wall) of the sheath 12. Figure 2 shows a gap between the outer wall 22 of the carrier element 20 and the inner wall 34 (the luminal wall) of the sheath 12, which appears purely for the sake of clarity in Figure 2. In practice, the carrier element 20 would be a close fit within the sheath 12 so as to have no gap or only a minimal gap between these two components.

The carrier element 20 is typically provided with one or more lumens running along the length of the element 20 (one of these lumens 26 being shown in Figure 2). As is convention in the art, there will typically be provided a guide wire lumen, a lumen for the provision of release mechanisms for releasing an implantable medical device carried proximate the distal end 13 of the introducer assembly 10, a lumen for flushing fluid and so on. The number and nature of the lumens provided within the carrier element 20 would be dependent upon the nature of the introducer assembly 10.

In the embodiment of Figure 2, the sheath element 12 is also provided with at least one strengthening element 28 which extends circumferentially around the sheath 12 and is embedded within the material of the sheath 12 (in this regard, the sheath 12 can be made of any conventional material known in the art).

The carrier element 20 includes (as is known in the art) a suitable location for holding an implantable medical device thereon and, typically, restraining elements such as restraining wires, restraining caps, as is considered necessary and desirable for that particular medical device.

The embodiment of Figure 2 thus provides a configuration or structure of sheath 12 and carrier element 20 which can be described as being flattened. In practice, this will cause the assembly to be more flexible when flexed about its flattened side (direction Y in Figure 2) and stiffer when flexed about its narrower sides (direction X shown in Figure 2). While this represents a departure from prior art introduce assemblies, which are designed to be round and to have the same flexibilities in all radial directions, it has been found that this difference in flexibilities improves trackability of the introducer assembly as this is fed through tortuous vessels. For instance, the structure 12, 20 will typically be more flexible in direction Y than a conventional introducer assembly for similar applications, even though being less flexible, potentially, in direction X. This varying flexibility enables a clinician to rotate the introducer assembly 10 during the deployment procedure in such a manner as to be able to assist in guiding the distal end 13 of the introducer assembly around curves and bends in a patient's vessels. Moreover, the distal end 13 of the introducer assembly will generally rotate when passed through a curve, so that the flattened sides of the structure will follow the curve, that is to generally orient so as to flex in direction Y around the curve. This provides a self-orienting bias to the assembly 10 in one or two orientations (these being 180 ° apart) and allowing the possibility for the clinician, should the device be 180° miss-oriented, to "flip" this by 180° and in practice to the desired orientation. Again with reference to Figure 2, it will be appreciated that the carrier element 20 cannot rotate relative to the sheath 12 as a result of their complimentary non-round shapes and in particular due to, in this embodiment, their oval shapes. As a result, any twisting of either the sheath 12 or the carrier element 20 will ensure equivalent twisting (rotation) of the other component. Furthermore, the torque strength of the carrier element 20 will in effect be added to the torque strength of the sheath 12, so as to give the overall structure increased torque strength compared to structures in which the sheath can rotate relative to the carrier element held therewithin. This is achieved without compromising the longitudinal flexibility of the introducer.

This complementary shape of carrier element and sheath extends, as described above, from the valve and handle unit 14 to the distal end 13 of the assembly 10 and in the preferred embodiment all the way to the dilator tip 21. In some embodiments, the dilator tip 21 has a shape at its proximal end (that is, the end which the use engages the distal end 13 of the sheath 12) which is complimentary to the internal oval wall 24 of the sheath 12. In other words, the proximal end of the dilator tip 21 has an oval shape which can register with the oval shape of the internal wall 24 of the sheath 12. In this manner, even the dilator tip 21 can be prevented from rotating relative to the sheath 12. The implantable medical device, which is carried at or proximate the distal end 13 of the assembly 10, will thus be maintained securely within the assembly 10 without being subjected to any relative twisting between the carrier element 20 and the sheath element 12.

Referring now to Figure 3 there is shown another embodiment of sheath assembly 12' (the carrier element 20 not being shown for the sake of clarity). In this embodiment, the sheath 12' is externally substantially round, that is the outer surface 30 of the sheath 12' is substantially circular in axial cross-section. The sheath 12' has an internal lumen wall 24 which is oval, as in the embodiment of Figure 2. A carrier element 20, such as that shown in Figure 2, can fit within the sheath 12'. The strengthening elements 28', described in further detail below, can be provided advantageously within the structure of the sheath element 12'. In this embodiment the strengthening elements 28' can be substantially circular in axial cross-section, as shown in Figure 3, and can be of the type found in existing sheath structures.

The embodiment of Figure 3 provides a sheath structure which has much closer flexibilities in all rotational directions, including in directions X and Y, compared to the embodiment of Figure 2. The embodiment of Figure 3 may be preferred in some instances, in dependence on the particular vasculature to which the introducer assembly is to be passed. Even though having a round cylindrical outer shape, this embodiment retains the feature of preventing rotation of the carrier element 20 relative to the sheath 12'.

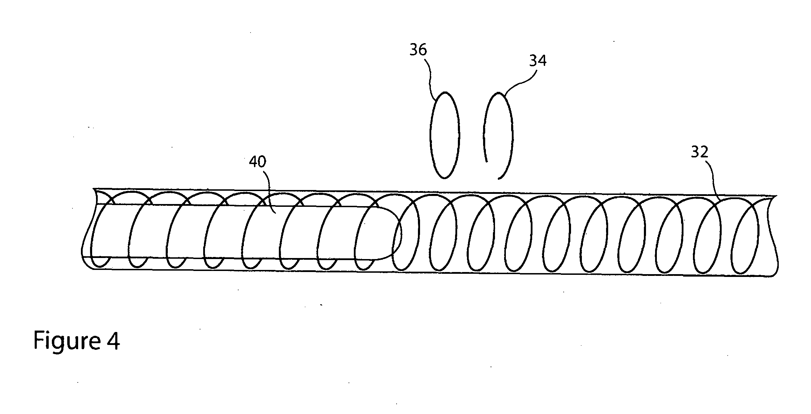

Referring now to Figure 4, there is shown schematically an embodiment of method for forming a sheath having the characteristics of the embodiments taught above.

As with many sheath assemblies, it is preferred that the sheath is provided with strengthening elements (28, 28' in the embodiments of Figures 2 and 3) to increase the kink resistance of the sheath and also to increase its pushability characteristics. In some embodiments, the strengthening elements could be metal braiding which is embedded within the walls of the sheath. In the embodiment shown in Figure 4, the strengthening elements include a coil 32, which is embedded within the structure of the sheath 12, 12' as shown in Figures 2 and 3. It is not excluded that a combination of coil and braiding might be used in some implementations.

With some structures of sheath, a strengthening coil can impart on the sheath different biasing forces in dependence upon the amount of twist which is imparted to the sheath. If the sheath is twisted in one direction, the coil will attempt to open, whereas if the sheath is twisted in another direction, the coil will tend to close. It some cases, this dynamic effect of the coil is not materially relevant to the functioning or characteristics of the sheath. In the preferred embodiments, however, the strengthening elements 28, 28' include not only one or more coils 32 but also stabilizing elements 34 and/or 36. The stabilizing elements 34 are in the form of an open ring which can be fitted between the turns of the coil 32. By contrast, the stabilizing elements 36 are closed rings which, likewise, can be located between turns of the coil 32. These elements 34, 36 act to stabilize the shape and diameter of the coil 32 during twisting of the sheath 12. Specifically, they will tend to retain their shape and diameter irrespective of the degree of twisting of the sheath and will thus tend to counter any tightening or widening of the coil. The effect will be to maintain the geometry of the sheath during its use.

With reference to Figure 4, the sheath can be formed by use of a mandrel 40 which has an external profile corresponding to the profile of the internal surface 24 of the sheath 12, 12', in other words which is oval in axial cross-section. Typically, over the mandrel 40 there will be provided a first layer of sheath material used to form the sheath 12 and over this is located the coil 32. In the case of use of stabilizing elements 34 of the open-ringed type, the mandrel 40 can be fed along the entirety of the length of the sheath 12 and therefore along the entirety of the length of the coil 32. The strengthening elements 34 can then be clipped onto the elements fitted on the mandrel 40, prior to the location of an outer layer of sheath material. The whole is then bonded, fused or otherwise cured so as to cause the layers of material to become essentially unitary in structure and thus to form the final sheath 12. Thus, once the sandwiched structure of layers of sheath material, coil and strengthening elements 34 are fitted to one another, the material is cured to form a unitary and integral sheath assembly. This formation process is well known in the art and therefore not described in detail herein.

In the case of use of strengthening elements 36 in the form of closed rings, these can be fitted to the assembly as the mandrel 40 is fed longitudinally through the turns of the coil 32. In an example, there may be provided a first layer of sheath material on the mandrel 40 which slides with the mandrel 40 through the turns of the coil 32, allowing the closed rings 36 to be fed over the mandrel and the inner layer of sheath material. Once the mandrel is in location with all the necessary strengthening rings 36 duly positioned, a second layer of sheath material is then applied over the assembly and then bonded, cured or heat set to form the final structure.

It will be apparent that it would be preferable to have a plurality of stabilizing elements 34, 36 spaced at intervals along the length of the coil 32 and thus along the length of the sheath 12. The number and relative spacings between the stabilizing elements 34, 36 will be dependent upon the characteristics of the sheath 12 and the application to which it is to be put.

It is to be understood that a combination of different stabilizing elements 34, 36 could be used in a sheath.

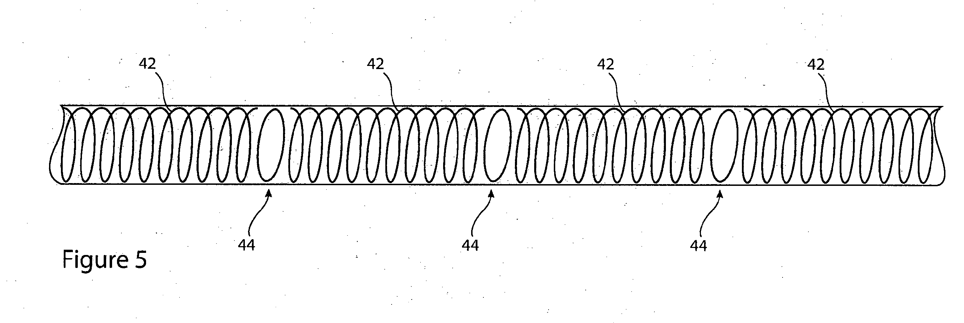

Figure 5 shows another embodiment of strengthening structure for the strengthening elements of 28, 28'. In this embodiment, there is provided a plurality of sections of strengthening coils 42. Interposed between adjacent coil sections 42 there are provided stabilizing elements 44 (which may be opened or closed rings, for example). This arrangement is relatively easier to manufacture (assemble) than the structure shown in Figure 4, whilst still ensuring that there are provided stabilizing elements 44 at regular intervals along the length of the sheath 12, 12'.

The strengthening elements 28, 28' can be made of any suitable materials and in particular any of the materials commonly used in the art. Examples include steel, Nitinol, other shape memory materials and so on.

Although the specific embodiments described above have a sheath and carrier element which have cooperating oval shapes, it is not necessary for them to be oval. Other shapes could be advantageous, such as part-circular, square or rectangular. It will be appreciated that in all such embodiments, the shapes would need to retain the flexibility of the introducer assembly 10. An oval shape is, however, preferred for the reasons described above.

## Claims

1. An introducer assembly (10) for deployment of a medical device or instrument, the introducer assembly including a flexible elongate outer sheath element- (12, 12') having proximal and distal ends; at least one flexible elongate carrier element (20) having proximal and distal ends located within the sheath element; the elongate outer sheath element being provided an internal lumen wall (24), which lumen wall has a non-round cross-section extending between substantially the proximal and distal ends of the sheath element; the elongate carrier element having an external walt surface (22) with a non-round cross-section, the internal lumen wall of the sheath element and the outer wall surface of the carrier element being of complimentary shapes, wherein the elongate outer sheath element (12, 12') and the elongate carrier element (20) are rotationally fixed relative to one another substantially along the entirety of their lengths between said proximal and distal ends;
wherein the corriplementary cross-sections of the sheath and carrier elements have a flattened configuration.

2. An assembly according to claim 1, wherein the internal lumenwall (24) of the sheath element (12, 12') and the outer wall surface (22) of the carrier element (20) have shapes which are continuous between said proximal and distal ends.

3. An assembly according to any preceding claim, wherein the internal lumen wall (24) of the sheath element (12, 12') is oval in transverse cross-section and the outer wall (22) of the carrier element (20) is oval in transverse cross-section.

4. An assembly according to any preceding claim, wherein the internal lumen wall (24) and the outer wall surface (22) are of complimentary shapes.

5. An assembly according to any preceding claim, wherein the carrier element (20) is a close fit within the lumen of the sheath element (12, 12').

6. An assembly according to any preceding claim, wherein the carrier element (20) is a carrier catheter or cannula having at its distal end a location for holding an implantable medical device.

7. An assembly according to any preceding claim, wherein the assembly has different flexibilities in different radial directions of bending.

8. An assembly according to any preceding claim, wherein the sheath element (12, 12') has a shape of an oval ring in axial cross-section.

9. An assembly according to any preceding claim, wherein the outer surface (22) of the carrier element (20) is oval in axial cross-section.

10. An assembly according to any preceding, including within the sheath element (12, 12') at least one strengthening element (28, 28'), 32, 34, 36, 42, 44) designed to maintain the non-round configuration of the sheath.

11. An assembly according to claim 10, wherein the strengthening element is non-round in axial cross-section of the sheath element (12, 12').

12. An assembly according to any of claims 10 and 11, wherein the strengthening element includes at least one coil (33, 42) and at least one stabilization ring (34, 36, 44).

13. An assembly according to any of claim 10-12, wherein the strengthening element is made of a metal or metal alloy.

14. An elongate outer sheath element (12, 12') for an introducer assembly (10), the outer sheath element having an internal lumen wall (24) which has an axial cross-section having a flattened configuration, there being provided within the sheath element at least one strengthening element (28, 28'), 32, 34, 36, 42, 44) designed to maintain the lumen wall in its flattened configuration.

15. A method of manufacturing an introducer assembly (10) as claimed in claim 10, the method including the steps of:
providing the at least one flexible elongate carrier element (20);
manufacturing the flexible elongate outer sheath element (12, 12'), wherein the method of manufacturing the flexible elongate outer sheath element (12, 12'), includes the steps of providing a mandrel which has an axial cross-section having a flattened configuration; locating at least one sheath tubing on the mandrel and fitting within the at least one tubing at least one strengthening element (28, 28', 32, 34, 36, 42, 44), which strengthening element adopts or has a flattened configuration in axial cross-section, which shape substantially corresponds to the axial cross-sectional configuration of the mandrel; and
locating the flexible elongate carrier element (20) within the flexible elongate outer sheath element (12, 12').

## Patentansprüche

1. Inserteranordnung (10) zum Einsetzen einer medizinischen Vorrichtung oder eines medizinischen Instruments, wobei die Inserteranordnung ein flexibles langgestrecktes Außenhüllenelement (12, 12') mit proximalen und distalen Enden; zumindest ein flexibles langgestrecktes Trägerelement (20) mit proximalen und distalen Enden in dem Hüllenelement aufweist; wobei das langgestreckte Außenhüllenelement mit einer inneren Lumenwand (24) ausgestattet ist, wobei die Lumenwand einen nicht-runden Querschnitt aufweist, der sich im Wesentlichen zwischen den proximalen und distalen Enden des Hüllenelements erstreckt; wobei das langgestreckte Trägerelement eine Außenwandfläche (22) mit einem nicht-runden Querschnitt aufweist, wobei die innere Lumenwand des Hüllenelements und die Außenwandfläche des Trägerelements komplementäre Formen aufweisen, worin das langgestreckte Außenhüllenelement (12, 12') und das langgestreckte Trägerelement (20) relativ zu einander im Wesentlichen entlang ihrer gesamten Längen zwischen den proximalen und distalen Enden gegen Drehung fixiert sind;
worin die komplementären Querschnitte der Hüllen- und Trägerelemente eine abgeflachte Konfiguration aufweisen.

2. Anordnung nach Anspruch 1, worin die innere Lumenwand (24) des Hüllenelements (12, 12') und die Außenwandfläche (22) des Trägerelements (20) Formen aufweisen, die zwischen den proximalen und distalen Enden kontinuierlich sind.

3. Anordnung nach einem der vorhergehenden Ansprüche, worin die innere Lumenwand (24) des Hüllenelements (12, 12') im transversalen Querschnitt oval ist und die Außenwand (22) des Trägerelements (20) im transversalen Querschnitt oval ist.

4. Anordnung nach einem der vorhergehenden Ansprüche, worin die innere Lumenwand (24) und die Außenwandfläche (22) komplementäre Formen aufweisen.

5. Anordnung nach einem der vorhergehenden Ansprüche, worin das Trägerelement (20) eng in dem Lumen des Hüllenelements (12, 12') sitzt.

6. Anordnung nach einem der vorhergehenden Ansprüche, worin das Trägerelement (20) ein Trägerkatheter oder eine Kanüle ist, dessen bzw. deren distalen Endes an einer Stelle zum Halten einer implantierbaren medizinischen Vorrichtung angeordnet ist.

7. Anordnung nach einem der vorhergehenden Ansprüche, worin die Anordnung unterschiedliche Flexibilitäten in verschiedenen radialen Biegerichtungen aufweist.

8. Anordnung nach einem der vorhergehenden Ansprüche, worin das Hüllenelement (12, 12') die Form eines ovalen Rings im axialen Querschnitt aufweist.

9. Anordnung nach einem der vorhergehenden Ansprüche, worin die Außenfläche (22) des Trägerelements (20) im axialen Querschnitt oval ist.

10. Anordnung nach einem der vorhergehenden Ansprüche, in dem Hüllenelement (12, 12') zumindest ein Verstärkungselement (28, 28', 32, 34, 36, 42, 44) aufweisend, das zur Aufrechterhaltung der nicht-runden Konfiguration der Hülle ausgelegt ist.

11. Anordnung nach Anspruch 10, worin das Verstärkungselement im axialen Querschnitt des Hüllenelements (12, 12') nicht-rund ist.

12. Anordnung nach einem der Ansprüche 10 und 11, worin das Verstärkungselement zumindest eine Spirale (33, 42) und zumindest einen Stabilisierungsring (34, 36, 44) aufweist.

13. Anordnung nach einem der Ansprüche 10-12, worin das Verstärkungselement aus einem Metall oder einer Metalllegierung gefertigt ist.

14. Langgestrecktes Hüllenelement (12, 12') für eine Inserteranordnung (10), wobei das Außenhüllenelement eine innere Lumenwand (24) mit einem axialen Querschnitt mit einer abgeflachten Konfiguration aufweist, wobei in dem Hüllenelement zumindest ein Verstärkungselement (28, 28', 32, 34, 36, 42, 44) bereitgestellt ist, das zur Aufrechterhaltung der Lumenwand in ihrer abgeflachten Konfiguration ausgelegt ist.

15. Verfahren zur Herstellung einer Inserteranordnung (10) nach Anspruch 10, worin das Verfahren die folgenden Schritte aufweist:
Bereitstellen des zumindest einen flexiblen langgestreckten Trägerelements (20);
Herstellen des flexiblen langgestreckten Außenhüllenelements (12, 12'), worin das Verfahren zur Herstellung des flexiblen langgestreckten Außenhüllenelements (12, 12') die Schritte des Bereitstellens eines Mandrins, der einen axialen Querschnitt mit einer abgeflachten Konfiguration aufweist; des Anordnens zumindest eines Hüllenschlauchs auf dem Mandrin und des Anbringens zumindest eines Verstärkungselements (28, 28', 32, 34, 36, 42, 44) in dem zumindest einen Schlauch umfasst, wobei das Verstärkungselement eine abgeflachte Konfiguration im axialen Querschnitt annimmt oder aufweist, wobei diese Form der axialen Querschnittskonfiguration des Mandrins entspricht; und
Anordnen des flexiblen langgestreckten Trägerelements (20) in dem flexiblen langgestreckten Außenhüllenelement (12, 12').

## Revendications

1. Ensemble d'introducteur (10) pour le déploiement d'un dispositif ou instrument médical, l'ensemble d'introducteur comportant un élément de gaine extérieur allongé flexible (12, 12') ayant des extrémités proximales et distales ; au moins un élément de support allongé flexible (20) ayant des extrémités proximales et distales situées à l'intérieur de l'élément de gaine ; l'élément de gaine extérieur allongé étant pourvu d'une paroi de lumière interne (24), laquelle paroi de lumière a une section transversale non ronde s'étendant entre substantiellement les extrémités proximales et distales de l'élément de gaine ; l'élément de support allongé ayant une surface de paroi externe (22) de section transversale non ronde, la paroi de lumière interne de l'élément de gaine et la surface de paroi externe de l'élément de support ayant des formes complémentaires, l'élément de gaine extérieur allongé (12, 12') et l'élément de support allongé (20) étant fixés à rotation l'un par rapport à l'autre substantiellement le long de toute leur longueur entre lesdites extrémités proximales et distales ;
Les sections transversales complémentaires des éléments de gaine et de support ayant une configuration aplatie.

2. Ensemble selon la revendication 1, dans lequel la paroi de lumière interne (24) de l'élément de gaine (12, 12') et la surface de paroi externe (22) de l'élément de support (20) ont des formes qui sont continues entre lesdites extrémités proximales et distales.

3. Ensemble selon l'une quelconque des revendications précédentes, dans lequel la paroi de lumière interne (24) de l'élément de gaine (12, 12') a une section transversale ovale et la paroi externe (22) de l'élément de support (20) a une section transversale ovale.

4. Ensemble selon l'une quelconque des revendications précédentes, dans lequel la paroi de lumière interne (24) et la surface de paroi externe (22) ont des formes complémentaires.

5. Ensemble selon l'une quelconque des revendications précédentes, dans lequel l'élément de support (20) est ajusté étroitement à l'intérieur de la lumière de l'élément de gaine (12, 12').

6. Ensemble selon l'une quelconque des revendications précédentes, dans lequel l'élément de support (20) est un cathéter ou une canule de support disposant à son extrémité distale d'un emplacement pour retenir un dispositif médical implantable.

7. Ensemble selon l'une quelconque des revendications précédentes, dans lequel l'ensemble présente différentes flexibilités dans différentes directions radiales de flexion.

8. Ensemble selon l'une quelconque des revendications précédentes, dans lequel l'élément de gaine (12, 12') a une forme de bague ovale en section transversale axiale.

9. Ensemble selon l'une quelconque des revendications précédentes, dans lequel la surface extérne (22) de l'élément de support (20) a une section transversale axiale ovale.

10. Ensemble selon l'une quelconque des revendications précédentes, comportant à l'intérieur de l'élément de gaine (12, 12'), au moins un élément de renforcement (28, 28', 32, 34, 36, 42, 44) conçu pour maintenir la configuration non ronde de la gaine.

11. Ensemble selon la revendication 10, dans lequel l'élément de renforcement n'est pas rond en une section transversale axiale de l'élément de gaine (12, 12').

12. Ensemble selon l'une quelconque des revendications 10 et 11, dans lequel l'élément de renforcement comporte au moins une bobine (33, 42) et au moins une bague de stabilisation (34, 36, 44).

13. Ensemble selon l'une quelconque des revendications 10 à 12, dans lequel l'élément de renforcement est fabriqué en métal ou en un alliage métallique.

14. Elément de gaine extérieur allongé (12, 12') pour un ensemble d'introducteur (10), l'élément de gaine extérieur ayant une paroi de lumière interne (24) qui a une section transversale axiale ayant une configuration aplatie, à l'intérieur de l'élément de gaine étant prévu au moins un élément de renforcement (28, 28', 32, 34, 36, 42, 44) conçu pour maintenir la paroi de la lumière dans sa configuration aplatie.

15. Procédé de fabrication d'un ensemble d'introducteur (10) selon la revendication 10, le procédé comportant les étapes suivantes :
fournir l'au moins un élément de support allongé flexible (20) ;
fabriquer l'élément de gaine extérieur allongé flexible (12, 12'), le procédé de fabrication de l'élément de gaine extérieur allongé flexible (12, 12') comportant les étapes consistant à fournir un mandrin qui a une section transversale axiale ayant une configuration aplatie ; positionner au moins un tubage de gaine sur le mandrin et ajuster à l'intérieur de l'au moins un tubage au moins un élément de renforcement (28, 28', 32, 34, 36, 42, 44), lequel élément de renforcement adopte ou présente une configuration aplatie en section transversale axiale, dont la forme correspond substantiellement à la configuration en section transversale axiale du mandrin ; et
positionner l'élément de support allongé flexible (20) à l'intérieur de l'élément de gaine extérieur allongé flexible (12, 12').
